Europäisches Patentamt

**European Patent Office**

**Office européen des brevets**

(11) Numéro de publication: **0 482 978 B1**

(12) **FASCICULE DE BREVET EUROPEEN**

(45) Date de publication de fascicule du brevet: **02.08.95** (51) Int. Cl.⁶: **A61N 1/368**

(21) Numéro de dépôt: **91402715.6**

(22) Date de dépôt: **11.10.91**

(54) Procédé de commande d'un stimulateur cardiaque en vue de la détection et le contrôle des tachycardies induites par conduction rétrograde du coeur.

(30) Priorité: **24.10.90 FR 9013199**

(43) Date de publication de la demande:
**29.04.92 Bulletin 92/18**

(45) Mention de la délivrance du brevet:
**02.08.95 Bulletin 95/31**

(84) Etats contractants désignés:
**BE CH DE ES GB IT LI LU SE**

(56) Documents cités:
**EP-A- 0 118 780**
**FR-A- 2 544 988**
**US-A- 4 569 350**

(73) Titulaire: **ELA MEDICAL**
**98, rue Maurice Arnoux**
**F-92120 Montrouge (FR)**

(72) Inventeur: **Limousin, Marcel**
**3, Rue Auber**
**F-92120 Montrouge (FR)**
Inventeur: **Nitsche, Rémi**
**55, Rue du Centre**
**F-78650 Beynes (FR)**

(74) Mandataire: **Laget, Jean-Loup et al**
**Cabinet Pierre Loyer**
**77, rue Boissière**
**F-75116 Paris (FR)**

Rank Xerox (UK) Business Services
(3.10/3.09/3.3.3)

## Description

La présente invention concerne d'une manière générale les stimulateurs cardiaques double chambre.

De tels stimulateurs sont adaptés afin de pouvoir stimuler l'oreillette et/ou le ventricule du coeur lorsque les dépolarisations ne se produisent pas de manière physiologique.

Le fonctionnement normal d'un coeur asservit une contraction du ventricule à une contraction de l'oreillette constatée par ce que les cardiologues appellent l'onde "p".

Dans certains cas, le coeur présente une conduction rétrograde qui propage vers l'oreillette l'onde due à la dépolarisation du ventricule et provoque la dépolarisation de ladite oreillette. Le stimulateur détecte cette dépolarisation et l'interprète comme une onde "p" habituelle.

A la fin du délai auriculo-ventriculaire le stimulateur stimule alors le ventricule en réponse à cette onde. La nouvelle dépolarisation du ventricule génère de nouveau une onde rétrograde qui est également mal interprétée et conduit à la stimulation du ventricule.

Le démarrage d'un tel cycle de fonctionnement est extrêmement dangereux car d'une part le ventricule peut se trouver stimulé à une fréquence trop élevée, et, d'autre part ledit ventricule est contracté alors qu'il n'a pas été complètement rempli de sang ce qui conduit à un débit sanguin insuffisant.

Une telle pathologie est appelée tachycardie induite par conduction rétrograde (TICR) et il est indispensable de réduire cette tachycardie.

A cet effet, il est nécessaire de différencier tout d'abord si l'accélération que l'on décèle dans le rythme cardiaque est due à une tachycardie sinusale ou à une telle tachycardie induite par conduction rétrograde (TICR).

Un moyen de différenciation connu par le document FR-2 544 988 consiste à réduire le délai auriculo-ventriculaire (délai AV ou DAV) s'écoulant entre la détection de l'onde "p" et la stimulation du ventricule en réponse à cette onde, puis, à mesurer le délai (appelé VP) entre ladite stimulation du ventricule et l'onde "p" suivante.

Lorsque l'on est en présence d'une tachycardie sinusale, l'accélération du rythme cardiaque est uniquement due à l'oreillette dont la fréquence augmente.

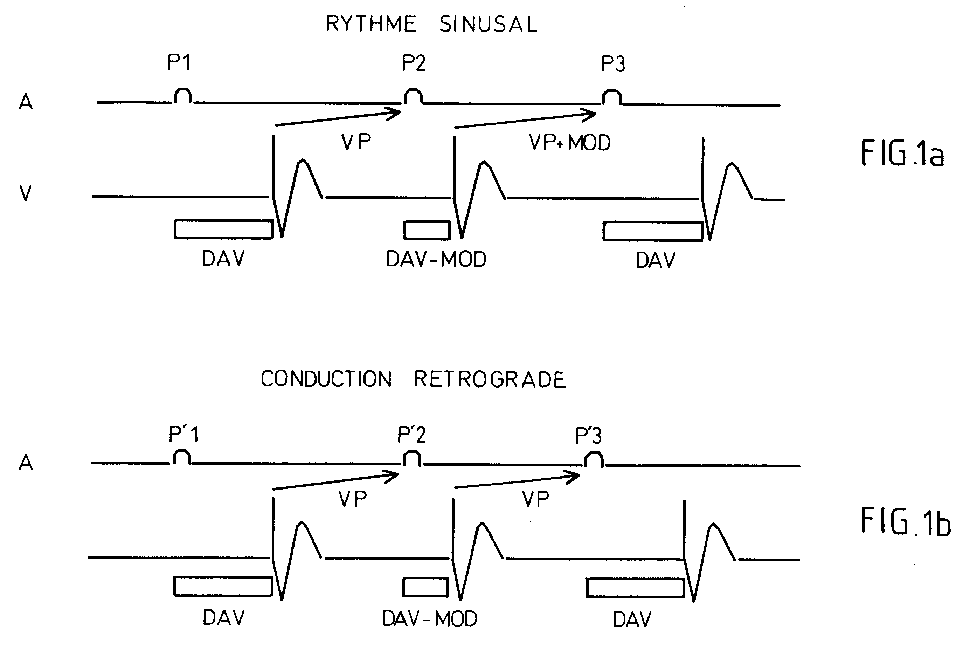

L'écart entre deux ondes "p" reste toujours le même, donc si l'on réduit le délai auriculo-ventriculaire, le délai VP augmente (Fig. 1a).

Par contre, lorsque l'accélération est due à une TICR, le ventricule pilote l'oreillette et quel que soit le délai AV, le délai VP reste stable (Fig. 1b).

Ce moyen de différenciation est déjà utilisé dans des stimulateurs cardiaques conformément au document FR-2 544 988, mais toujours dans des procédures de contrôle parfois trop longues pour le patient, car elles risquent d'entretenir la TICR trop longtemps.

La présente invention concerne donc un nouveau procédé de contrôle des TICR qui permet de les détecter plus rapidement, et donc de les réduire beaucoup plus tôt.

Les TICR étant dues à des dysfonctionnements ou inadéquations du programme du stimulateur à l'état du coeur, le procédé selon l'invention tend également à reprogrammer rapidement le stimulateur pour éviter leur reproduction trop fréquente.

La présente invention a pour objet un procédé de commande d'un stimulateur cardiaque en vue de la détection et le contrôle des tachycardies induites par conduction rétrograde (TICR) du coeur, dans lequel on apprécie le risque d'installation d'une TICR par le fait que le délai VP entre la stimulation du ventricule et la détection d'une onde "p" est inférieur à une valeur comprise entre 400 et 500 ms et de préférence inférieur à 450 ms, caractérisé en ce que :

- lorsque le délai VP est inférieur à 450 ms, on mesure ce délai VP sur un nombre de cycles cardiaques compris entre 2 et 16 et de préférence égal à 8,
- on calcule la différence entre la plus petite et la plus grande des valeurs du délai VP,
- on compare cette différence à une première valeur de seuil, le risque de TICR étant exclu si ladite différence est supérieure à ladite première valeur de seuil,
- dans le cas où ladite différence est inférieure à la première valeur de seuil, on la compare à une deuxième valeur de seuil inférieure à la première,
- dans le cas où ladite différence est inférieure à ladite deuxième valeur de seuil, on module le délai auriculoventriculaire (DAV) d'une première durée et on mesure le délai VP,
   . si le délai VP a varié par rapport aux précédents d'une quantité inférieure à ladite deuxième valeur de seuil, on est en présence d'une TICR et on la réduit,
   . si le délai VP a varié par rapport aux précédents d'une quantité comprise entre lesdites première et deuxième valeurs de seuil, on module le délai AV d'une deuxième durée supérieure à ladite première durée et on mesure le délai VP,
      . si le délai VP a varié par rapport aux précédents d'une quantité inférieure à ladite première valeur de seuil, on est en présence d'une TICR et on la réduit,
      . si le délai VP a varié d'une quantité supérieure à ladite première valeur de seuil, on n'est pas en présence d'une TICR,

- et dans le cas où ladite différence est supérieure à ladite deuxième valeur de seuil, on module le délai AV de ladite deuxième durée et on procède comme précédemment.

Selon d'autres caractéristiques de l'invention :

- la première valeur de seuil est comprise entre 25 et 40 ms et de préférence égale à 31 ms,
- la deuxième valeur de seuil est comprise entre 10 et 25 ms et de préférence égale à 16 ms,
- la première durée de modulation du délai AV est comprise entre 40 et 55 ms et de préférence égale à 47 ms,
- la deuxième durée de modulation du délai AV est comprise entre 55 et 70 ms et de préférence égale à 63 ms,
- lorsque le délai VP entre la stimulation du ventricule et la détection d'une onde "p" est inférieur à 450 ms, l'on mesure les délais VP d'un nombre de cycles cardiaques consécutifs compris entre 2 et 16 et de préférence égal à 8, et l'on calcule la différence entre le plus grand et le plus petit de ces délais, que l'on compare à une valeur de seuil égale de préférence à 31 ms,
- lorsque ladite différence calculée est supérieure à 31 ms, l'on détermine qu'il n'y a pas de TICR,
- lorsque ladite différence calculée est inférieure à 31 ms, donc lorsque VP est dit stable, l'on compare ladite différence à 16 ms,
- lorsque ladite différence calculée est inférieure à 16 ms, on module le délai auriculo-ventriculaire de 47 ms sur un cycle, puis, si VP est stable à 16 ms on détermine qu'il y a TICR et on la réduit, tandis que si VP n'est pas stable, on module le délai auriculo-ventriculaire à 63ms sur un cycle cardiaque,
- lorsque ladite différence calculée est supérieure à 16 ms, on module le délai auriculo-ventriculaire à 63 ms sur un cycle cardiaque,
- après la modulation à 63 ms, si VP est stable à 31 ms, on détermine qu'il y a TICR et on la réduit, tandis que si VP n'est pas stable, on détermine qu'il y a tachycardie sinusale,
- lorsque l'on a déterminé qu'il y avait tachycardie sinusale, l'on met en route un compteur, afin de ne pas reprendre le procédé de contrôle avant un nombre de cycles cardiaques compris entre 50 et 150 et de préférence égal à 100.
- la modulation du délai auriculo-ventriculaire est négative, ou bien positive lorsque la modulation négative implique une fréquence de stimulation ventriculaire supérieure à la fréquence maximale programmée ou un délai AV trop court,
- l'on comptabilise comme TICR, toute confirmation de la stabilité du délai VP, ainsi que la détection d'un bloc rétrograde,
- la détection d'un bloc rétrograde est avérée lorsque le temps VP au cours du dixième cycle est supérieur à une valeur (VP max + $\|Mod\|$ + Ds),
- lorsque l'on a comptabilisé un nombre prédéterminé, de l'ordre de 5, de TICR en une journée, on initialise une reprogrammation du stimulateur cardiaque.

Pour permettre de mieux expliciter l'invention, on a représenté au dessin annexé :

Figure 1a - une représentation schématique des enregistrements de l'oreillette (A) et du ventricule (V) en cas de rythme cardiaque sinusal,

Figure 1b - une représentation schématique des mêmes enregistrements en cas de conduction rétrograde, correspondant à une TICR,

Figure 2a - une représentation schématique de la stimulation cardiaque en cas de rythme sinusal,

Figure 2b - une représentation schématique de la stimulation cardiaque en cas de conduction rétrograde correspondant à une TICR,

Figure 2c - une représentation schématique de la stimulation cardiaque en cas d'apparition d'un bloc rétrograde induit par la stimulation du ventricule,

Figure 3 - un schéma-bloc des différentes opérations correspondant au procédé selon l'invention, de détection et de contrôle des TICR, dans le cas d'une phase de détection de 8 cycles cardiaques.

On sait qu'il ne peut pas se produire de TICR soit lorsque l'oreillette est stimulée par le stimulateur cardiaque, soit lorsque le ventricule se contracte spontanément, soit lorsque le rythme cardiaque est lent. Donc, le procédé ne sera pas initialisé lorsque l'une de ces conditions est remplie.

On sait également que lorsque le délai VP est supérieur à 450 millisecondes (ms) il ne peut se produire de TICR.

Lorsque le délai VP devient inférieur à 400 à 500 ms et de préférence à 450 ms, on considère qu'il existe un risque d'être en présence d'une TICR.

Afin de contrôler le délai VP, le procédé selon l'invention consiste à observer un nombre de cycles consécutifs compris entre 2 et 16 et de préférence égal à 8.

Au cours de ces cycles, on mesure les délais VP et on calcule la différence entre le plus grand (VP max) et le plus petit (VP min) de ces délais.

Dans le cas où cette différence est supérieure à 25 à 40 ms et de préférence à 31 ms on peut dire que VP n'est pas stable et donc que l'on n'a pas affaire à une TICR mais à une tachycardie sinusale, donc physiologique.

Par contre, si l'on constate que VP est stable selon le critère des 31 ms, c'est-à-dire que la différence entre la plus grande et la plus petite des valeurs est inférieure ou égale à 25 à 40 ms et de préférence à 31 ms, alors il y a un risque de TICR.

Le procédé selon l'invention distingue alors deux cas :

- la différence entre les VP est inférieure à 31 ms ainsi qu'à 10 à 25 ms et de préférence 16 ms ;
- la différence entre les VP est comprise entre 31 et 16ms.

Dans le premier cas, on module sur un cycle cardiaque à 40 à 55ms et de préférence à 47ms, c'est-à-dire que l'on réduit ou augmente le DAV de 47ms et on observe VP en comparaison avec les derniers VP mesurés. La modulation est positive si la modulation négative conduit à dépasser la fréquence maximale programmée, ou conduit à un délai AV trop court (< 31 ms) ou négatif.

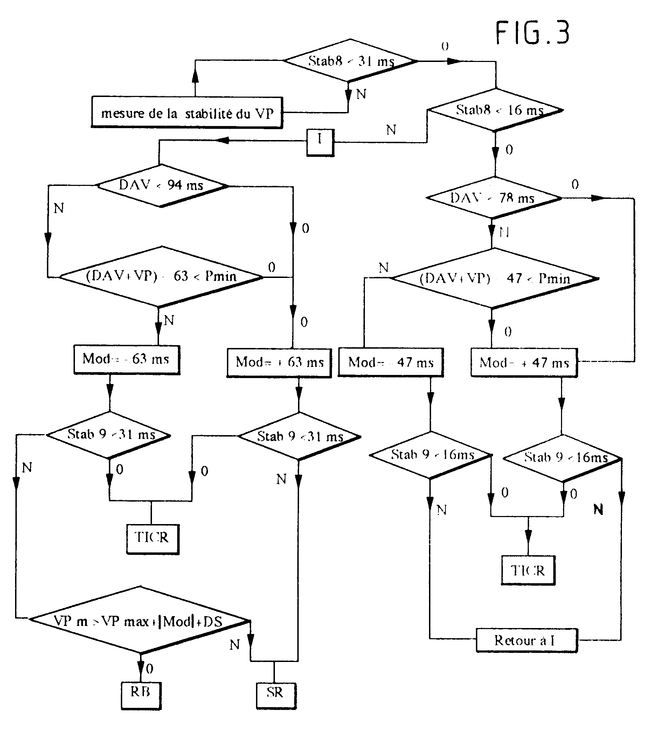

Si VP est stable, c'est-à-dire qu'il a varié de moins de 16 ms, alors on est sûr d'être en présence d'une TICR que l'on réduit de manière connue en soi (case TICR de droite, Fig 3).

Si VP n'est pas stable, c'est-à-dire qu'il a varié de plus de 16 ms, alors on module un cycle (le neuvième) à 55 à 70 ms et de préférence à 63 ms et on examine la stabilité par rapport à 31 ms.

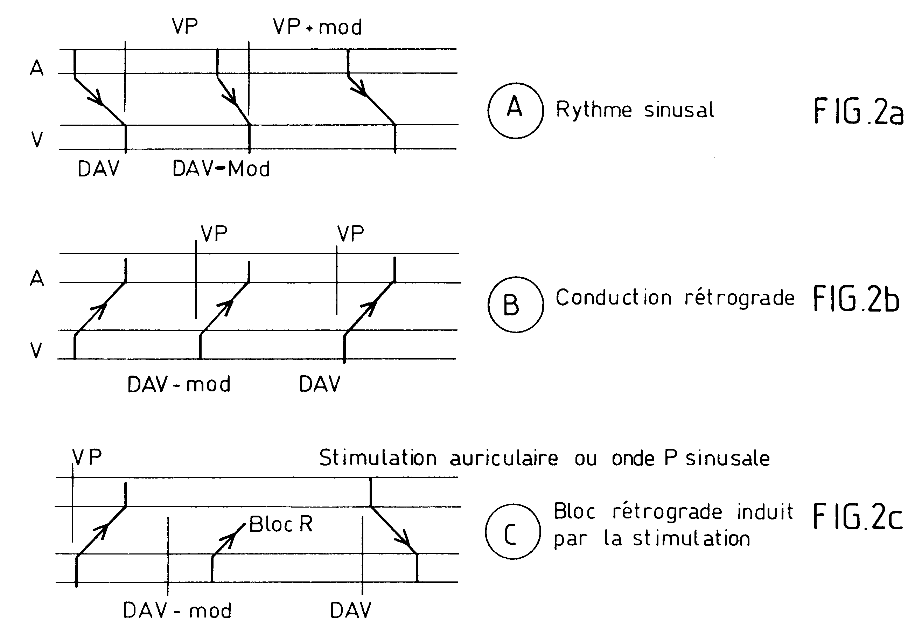

Lorsque VP n'est pas stable (case SR, Fig 3), on met en route un compteur afin de ne pas remettre en action le procédé avant un nombre de cycles cardiaques compris entre 50 et 150 et de préférence égal à 100, car la tachycardie est sinusale et les modulations mises en oeuvre par le procédé sont inutiles (Fig. 2a).

Lorsque VP est stable (case TICR de gauche, Fig 3), on est en présence d'une TICR (Fig 2b) que l'on réduit.

Dans le second cas c'est-à-dire celui dans lequel la différence des VP sur huit cycles est comprise entre 31 et 16 ms, on module un cycle à 63 ms et on examine la stabilité par rapport à 31 ms.

Les conclusions sont alors les mêmes que ci-dessus c'est-à-dire que la tachycardie est sinusale si VP n'est pas stable, ce qui déclenche la mise en route du compteur afin de ne pas remettre en action le procédé avant un nombre de cycles cardiaques, compris entre 50 et 150 et de préférence égal à 100, et que l'on est en présence d'une TICR si VP est stable.

Lorsque le temps VP après le cycle de modulation du délai AV est mesuré stable, il faut réduire la tachycardie. Dans le cycle suivant, une période réfractaire auriculaire de 450 ms évite de faire démarrer un délai AV sur l'onde "p" rétrograde. A la fin de l'intervalle d'échappement, l'oreillette est stimulée, puis le ventricule après le délai AV : la synchronisation est ainsi rétablie.

Le procédé selon l'invention permet, comme on peut le constater, de déterminer après 10 cycles cardiaques s'il y a ou non TICR tandis que les procédés connus nécessitent souvent une vingtaine ou même plus de trente cycles cardiaques pour confirmer ladite TICR.

Un autre avantage du procédé selon l'invention réside dans sa méthode de comptage des TICR.

En effet, on comptabilise comme TICR toute confirmation de la stabilité du délai VP, ainsi que l'apparition d'un bloc de conduction rétrograde.

Cela est dû à la constatation que dans certains cas, la réduction du délai auriculo-ventriculaire, en augmentant la fréquence du ventricule, bloque la conduction rétrograde et casse immédiatement la TICR (Fig. 2c), ou la ralentit.

Les TICR de ce type ne sont pas identifiées par les procédés de contrôle connus, ce qui peut s'avérer dangereux pour le porteur du stimulateur.

Le procédé de détection et de contrôle selon l'invention prévoit la détection des blocs rétrogrades. Lorsque, au cours du neuvième cycle, on a constaté que VP n'est pas stable par rapport à 31 ms, on le compare à la valeur (VP max + ‖Mod‖ + Ds), dans laquelle VP max est la plus grande valeur mesurée de VP au cours des huit cycles précédents, ‖Mod‖ est la valeur absolue de la modulation du DAV, et Ds est une durée de sécurité comprise entre 50 et 150 ms et de préférence égale à 100 ms.

Si VP m, temps VP au cours du dixième cycle, est supérieur à cette valeur, on est en présence d'un bloc rétrograde (case RB de la Fig 3) et on le comptabilise comme une TICR, dans un compteur particulier.

Chaque fois que ce compteur est arrivé à un nombre déterminé, par exemple 5, sur une journée, le procédé de contrôle selon l'invention initialise une reprogrammation du stimulateur tendant à modifier les paramètres afin d'éviter de générer une nouvelle TICR. Une telle méthode de comptage permet de ne pas laisser de côté de TICR, et de reprogrammer très rapidement le stimulateur avant que la situation ne risque de devenir grave.

Le procédé objet de l'invention a été décrit comme utilisant des réductions du délai auriculo-ventriculaire. On ne sortira pas du cadre de la présente invention si l'on augmente ledit délai auriculo-ventriculaire, car la détermination de la TICR par la stabilité de VP n'est alors nullement remise en cause.

Il faut donc comprendre que les modulations évoquées plus haut sont des variations en plus ou en moins.

Les variations négatives seront préférées chaque fois qu'elles seront possibles puisqu'elles favorisent la réduction d'une TICR.

Les variations positives seront quant à elles, mises en oeuvre lorsque la diminution du DAV risquerait d'amener une valeur inférieure à celle fixée dans le stimulateur, voire négative, ou lorsque la fréquence de stimulation ventriculaire risquerait de dépasser la fréquence maximale de stimulation ventriculaire autorisée (ou période minimale de stimulation ventriculaire).

Sur la figure 3, les abréviations correspondent à :

| | |
|---|---|
| DAV | : délai auriculo-ventriculaire ; |
| VP | : temps de conduction rétrograde ; |
| Stab 8 | : stabilité du temps VP durant les 8 cycles de détection ; |
| Mod | : modulation du DAV ; |
| Stab 9 | : stabilité du temps VP durant les phases de détection et de confirmation (i.e. 9 cycles pour la première modulation du DAV); |
| VPm | : temps VP dans le cycle suivant la modulation du DAV ; |
| SR | : rythme sinusal ; |
| RB | : bloc rétrograde induit par une réduction du DAV ; |
| VP max | : temps VP maximal durant la phase de détection ; |
| Pmin | : période correspondant à la fréquence maximale admissible pour le coeur ; |
| TICR | : tachycardie induite par conduction rétrograde ; |
| Ds | : Durée de sécurité. |

## Revendications

1. Procédé commande d'un stimulateur cardiaque en vue de la détection et le contrôle des tachycardies induites par conduction rétrograde (TICR) du coeur, dans lequel on apprécie le risque d'installation d'une TICR par le fait que le délai VP entre la stimulation du ventricule et la détection d'une onde "p" est inférieur à 400 à 500 ms et de préférence à 450 ms, caractérisé en ce que
   - lorsque le délai VP est inférieur à 450 ms, on mesure ce délai VP sur un nombre de cycles cardiaques, compris entre 2 et 16 et de préférence égal à 8,
   - on calcule la différence entre la plus petite et la plus grande des valeurs du délai VP,
   - on compare cette différence à une première valeur de seuil, le risque de TICR étant exclu si ladite différence est supérieure à ladite première valeur de seuil,

   - dans le cas où ladite différence est inférieure à la première valeur de seuil, on la compare à une deuxième valeur de seuil inférieure à la première,
   - dans le cas où ladite différence est inférieure à ladite deuxième valeur de seuil, on module le délai auriculoventriculaire (DAV) d'une première durée et on mesure le délai VP,
   . si le délai VP a varié par rapport aux précédents d'une quantité inférieure à ladite deuxième valeur de seuil, on est en présence d'une TICR,
   . si le délai VP a varié par rapport aux précédents d'une quantité comprise entre lesdites première et deuxième valeurs de seuil, on module le délai AV d'une deuxième durée supérieure à ladite première durée et on mesure le délai VP,
   . si le délai VP a varié par rapport aux précédents d'une quantité inférieure à ladite première valeur de seuil, on est en présence d'une TICR,
   . si le délai VP a varié d'une quantité supérieure à ladite première valeur de seuil, on n'est pas en présence d'une TICR,
   - et dans le cas où ladite différence est supérieure à ladite deuxième valeur de seuil, on module le délai AV de ladite deuxième durée et on procède comme précédemment.

2. Procédé selon la revendication 1 caractérisé en ce que la première valeur de seuil est comprise entre 25 et 40 ms et de préférence de 31 ms,

3. Procédé selon la revendication 1 caractérisé en ce que la deuxième valeur de seuil est comprise entre 10 et 25 ms et de préférence de 16 ms,

4. Procédé selon la revendication 1 caractérisé en ce que la première durée de modulation du délai AV est comprise entre 40 et 55 ms et de préférence de 47 ms,

5. Procédé selon la revendication 1 caractérisé en ce que la deuxième durée de modulation du délai AV est comprise entre 55 et 70 ms et de préférence de 63 ms,

6. Procédé selon la revendication 5, caractérisé en ce que, après la modulation à 63 ms, si VP est stable à 31 ms, on détermine qu'il y a TICR, tandis que si VP n'est pas stable, on détermine qu'il y a tachycardie sinusale.

**7.** Procédé selon la revendication 6, caractérisé en ce que, lorsque l'on a déterminé qu'il y avait tachycardie sinusale, l'on met en route un compteur, afin de ne pas reprendre le procédé de contrôle avant un nombre de cycles cardiaques compris entre 50 et 150 et de préférence égal à 100.

**8.** Procédé selon l'une quelconque des revendications précédentes, caractérisé en ce que la modulation du délai auriculo-ventriculaire est négative, ou bien positive lorsque la modulation négative implique une fréquence de stimulation ventriculaire supérieure à la fréquence maximale programmée ou un délai AV trop court.

**9.** Procédé selon l'une quelconque des revendications précédentes, caractérisé en ce que l'on comptabilise comme TICR, toute confirmation de la stabilité du délai VP, ainsi que la détection d'un bloc rétrograde.

**10.** Procédé selon la revendication 9, caractérisé en ce que la détection d'un bloc rétrograde est avérée lorsque le temps VP au cours du dixième cycle est supérieur à une valeur (VP max + ‖Mod‖ + Ds), dans laquelle ‖Mod‖ est la valeur absolue de la modulation du DAV, et Ds est une durée de sécurité comprise entre 50 et 150ms et de préférence égale à 100ms.

**11.** Procédé selon la revendication 9, caractérisé en ce que lorsque l'on a comptabilisé un nombre prédéterminé, de l'ordre de 5, de TICR en une journée, on initialise une reprogrammation du stimulateur cardiaque.

**Claims**

**1.** Method for controlling a cardiac pacemaker with a view to detect and control endless loop tachycardias (ELT) of a heart, wherein the risk of an ELT becoming established is appreciated through the fact that the VP time interval between the ventricle pacing and the detection of a P wave is shorter than a value comprised between 400 and 500 milliseconds (ms) and preferably less than 450 ms, characterized in that :
- when the VP interval is less than 450 ms, a measurement is made of this VP interval over a number of heart cycles comprised between 2 and 16, and preferably equal to 8,
- a calculation is made of the difference between the smallest and the largest of the values of the VP time interval,

- this difference is compared with a first threshold value, the risk of an ELT being excluded if the said difference is larger than the said first threshold value,
- in case the said difference is less than the first threshold value, it is compared to a second threshold value which is smaller than the first threshold value,
- in case the said difference is less than the said second threshold value, the AVD is modulated with a first duration and the following VP time interval is measured,
  . if the VP time interval has varied relatively to the preceding ones by a quantity less than the said second threshold value, this means that an ELT is occurring,
  . if the VP time interval has varied relatively to the preceding ones by a quantity comprised between said first and second threshold values, the AVD is then modulated with a second duration being longer than said first duration, and the VP time interval is measured,
  . if the VP time interval has varied relatively to the preceding ones by a quantity less than said first threshold value, this means that an ELT is occurring,
  . if the VP time interval has varied by a quantity larger than said first threshold value, this means that no ELT is present,
- and in case the said difference is larger than the said second threshold value, the AVD is modulated with the said second duration, and one proceeds in the same manner as previously.

**2.** Method according to claim 1, characterized in that the first threshold value is included within 25 and 40 ms and preferably equal to 31 ms.

**3.** Method according to claim 1, characterized in that the second threshold value is included within 10 and 25 ms and preferably equal to 16 ms.

**4.** Method according to claim 1, characterized in that the first duration of modulation of the AVD is included between 40 and 55 ms and preferably equal to 47 ms.

**5.** Method according to claim 1, characterized in that the second duration of modulation of the AVD is included between 55 and 70 ms and preferably equal to 63 ms.

**6.** Method according to claim 5 characterized in that after modulating at 63 ms, if VP is stable at 31 ms, it is determined that an ELT is present, whereas if VP is not stable, it is determined that there is a sinus tachycardia.

7.  Method according to claim 6 characterized in that when it has been determined that there was a sinus tachycardia, a counter is started in order that the control process will not be resumed before a number of cardiac cycles comprised between 50 and 150, preferably equal to 100.

8.  Method according to anyone of the preceding claims characterized in that the modulation of the AVD is negative, or else positive when the negative modulation implies either a ventricular pacing rate higher than the programmed upper rate, or a too short AVD.

9.  Method according to anyone of the preceding claims characterized in that an account is kept, as ELT, of every confirmation of the stability of the VP time interval, as well as of the detection of a retrograde block.

10. Method according to claim 9 characterized in that the detection of a retrograde block is ascertained when the VP time interval during the tenth cycle is longer than a (VP max + $\|Mod\|$ + Ds) value, in which $\|Mod\|$ is the absolute value of the modulation of the AVD, and Ds is a safety duration included within 50 and 150 ms and preferably equal to 100 ms.

11. Method according to claim 9 characterized in that when there has been recorded a predetermined number, that is of the order of 5, of ELT during one day, a reprogramming of the cardiac pulse generator is initialized.

**Patentansprüche**

1.  Verfahren zum Betreiben eines Herzschrittmachers im Hinblick auf die Bestimmung und Steuerung von durch retrograde Überleitung induzierten Tachykardien (TICR) des Herzes, in welchem man das Risiko des Eintretens einer TICR aufgrund der Tatsache beurteilt, daß die VP-Verzögerung zwischen der Stimulierung des Ventrikels und der Erfassung einer P-Zacke kleiner als 400 bis 500 ms und vorzugsweise 450 ms ist,
    **dadurch gekennzeichnet, daß**
    - wenn die VP-Verzögerung kleiner als 450 ms ist, diese VP-Verzögerung über eine Anzahl von Herz-Zyklen hinweg gemessen wird, die zwischen 2 und 16 liegt und vorzugsweise gleich 8 ist,

    - die Differenz zwischen dem kleinsten und dem größten der Werte der VP-Verzögerung berechnet wird,
    - diese Differenz mit einem ersten Schwellenwert verglichen wird, wobei das Risiko einer TICR ausgeschlossen wird, wenn die Differenz größer als der erste Schwellenwert ist,
    - in dem Fall, in dem die Differenz kleiner als der erste Schwellenwert ist, diese mit einem zweiten Schwellenwert verglichen wird, der kleiner als der erste ist,
    - in dem Fall, in dem die Differenz kleiner als der zweite Schwellenwert ist, die aurikulär-ventrikuläre Verzögerung (DAV) mit einer ersten Dauer moduliert wird und die VP-Verzögerung gemessen wird,
    . wenn die VP-Verzögerung sich mit Bezug auf die vorhergehenden um einen Betrag verändert hat, der kleiner ist als der zweite Schwellenwert, eine TICR vorliegt,
    . wenn die VP-Verzögerung sich mit Bezug auf die vorhergehenden um einen Betrag verändert hat, der zwischen dem ersten und dem zweiten Schwellenwert liegt, die AV-Verzögerung mit einer zweiten Dauer moduliert wird, die größer ist als die erste Dauer, und die VP-Verzögerung gemessen wird,
        . wenn die VP-Verzögerung sich mit Bezug auf die vorhergehenden um einen Betrag verändert hat, der kleiner ist als der erste Schwellenwert, eine TICR vorliegt,
        . wenn die VP-Verzögerung sich um einen Betrag verändert hat, der größer ist als der erste Schwellenwert, keine TICR vorliegt,
    - und in dem Fall, in dem die Differenz größer ist als der zweite Schwellenwert, die AV-Verzögerung mit der zweiten Dauer moduliert wird und wie vorhergehend verfahren wird.

2.  Verfahren nach Anspruch 1,
    **dadurch gekennzeichnet, daß**
    der erste Schwellenwert zwischen 25 und 40 ms liegt und vorzugsweise 31 ms beträgt.

3.  Verfahren nach Anspruch 1,
    **dadurch gekennzeichnet, daß**
    der zweite Schwellenwert zwischen 10 und 25 ms liegt und vorzugsweise 16 ms beträgt.

4.  Verfahren nach Anspruch 1,
    **dadurch gekennzeichnet, daß**
    die erste Modulationsdauer der AV-Verzögerung zwischen 40 und 55 ms liegt und vorzugsweise 47 ms beträgt.

**5.** Verfahren nach Anspruch 1,
**dadurch gekennzeichnet, daß**
die zweite Modulationsdauer der AV-Verzögerung zwischen 55 und 70 ms liegt und vorzugsweise 63 ms beträgt.

**6.** Verfahren nach Anspruch 5,
**dadurch gekennzeichnet, daß**
nach der Modulation mit 63 ms, wenn VP 31 ms stabil ist, bestimmt wird, daß eine TICR vorliegt, während, wenn VP nicht stabil ist, bestimmt wird, daß eine Sinus-Tachykardie vorliegt.

**7.** Verfahren nach Anspruch 6,
**dadurch gekennzeichnet, daß,**
wenn bestimmt wurde, daß eine Sinus-Tachykardie vorlag, ein Zähler ausgelöst wird, um den Steuerungsvorgang nicht vor einer Anzahl von Herz-Zyklen wiederaufzunehmen, die zwischen 50 und 150 liegt und vorzugsweise gleich 100 ist.

**8.** Verfahren nach einem der vorhergehende Ansprüche,
**dadurch gekennzeichnet, daß**
die Modulation der aurikulär-ventrikulären Verzögerung negativ ist, oder positiv ist, wenn die negative Modulation eine ventrikuläre Stimulationsfrequenz, die größer als die programmierte maximale Frequenz ist, oder eine zu kurze AV-Verzögerung impliziert.

**9.** Verfahren nach einem der vorhergehenden Ansprüche,
**dadurch gekennzeichnet, daß**
jede Bestätigung der Stabilität der VP-Verzögerung, ebenso wie die Erfassung eines retrograden Blockes, als eine TICR registriert wird.

**10.** Verfahren gemäß Anspruch 9,
**dadurch gekennzeichnet, daß**
die Erfassung eines retrograden Blockes sich als wahr erweist, wenn die Zeit VP im Verlauf des zehnten Zyklus größer als ein Wert (VP max + ‖Mod‖ + Ds) ist, wobei ‖Mod‖ der Absolutwert der Modulation der DAV ist, und Ds eine zwischen 50 und 150 ms liegende und vorzugsweise gleich 100 ms betragende Sicherheitsdauer ist.

**11.** Verfahren nach Anspruch 9,
**dadurch gekennzeichnet, daß,**
wenn während eines Tages eine vorbestimmte Anzahl, in der Größenordnung von 5, von TICR registriert wurden, eine Neuprogrammierung des Herzschrittmachers initialisiert wird.

RYTHME SINUSAL

A

V

P1  P2  P3

VP  VP+MOD

DAV  DAV-MOD  DAV

FIG.1a

CONDUCTION RETROGRADE

A

V

P'1  P'2  P'3

VP  VP

DAV  DAV-MOD  DAV

FIG.1b

EP 0 482 978 B1

(A) Rythme sinusal **FIG.2a**

(B) Conduction rétrograde **FIG.2b**

(C) Bloc rétrograde induit par la stimulation **FIG.2c**

EP 0 482 978 B1

# FIG.3